# EUROPEAN PATENT SPECIFICATION

(11) **EP 3 595 650 B1**
(45) Date of publication and mention of the grant of the patent: **28.04.2021**
(21) Application number: 18717420.6
(22) Date of filing: 13.03.2018
(51) Int. Cl.: A61K 31/352, A61K 31/575, A61K 9/06, A61K 47/14, A61P 43/00

(54) **COMPOSITION FOR USE IN THE REDUCTION OF HAIR GROWTH**
ZUSAMMENSETZUNG ZUR REDUZIERUNG DES HAARWACHSTUMS
COMPOSITION POUR LA RÉDUCTION DE LA POUSSE DES CHEVEUX

(30) Priority: 13.03.2017 IT 201700027628
(43) Date of publication of application: 22.01.2020
(73) Proprietor: Neilos S.r.l., 80063 Piano di Sorrento (NA) (IT)
(72) Inventor: DI MAIO, Umberto, 80063 Piano di Sorrento (NA) (IT)
(74) Representative: Hoffmann Eitle
(86) International application number: PCT/IB2018/051651
(87) International publication number: WO 2018/167657

(56) References cited:
- EP-A1- 1 314 438
- EP-A2- 1 074 240
- WO-A1-2012/131638
- WO-A2-01/03687
- WO-A2-2009/052518
- US-A1- 2005 037 099

## Description

### FIELD OF THE INVENTION

The present invention relates to a pharmaceutical or cosmetic composition for reducing the rate of hair growth and for the prevention and/or treatment of hirsutism.

### DESCRIPTION

The hair follicle is a very complex organ, consisting of different cell populations and characterised by different locations and different expressions of cell components. It thus represents the only system of its kind, very dynamic and characterised by a growth cycle that continues throughout life. The growth cycle is under the control of different hormones and local growth factors, which are probably produced both by the follicle cells and the cells of the follicular papilla.

Hirsutism is a condition characterised by an increased growth of body hair, due to the increased sensitivity of hair follicles to androgens or by an increase in the synthesis of these hormones. Androgens lead to an increase in hair follicle size, shaft diameter and duration of the anagen phase, and an increase in the secretion of sebum from the sebaceous glands. All these aspects are often persistent in hirsutism. In most cases, hirsutism derives from a benign cause, which may be polycystic ovary syndrome (PCSO) or idiopathic hirsutism, observed despite low basal levels of androgens in the blood. Other rarer causes of hirsutism are congenital adrenal hyperplasia, Cushing's syndrome and ovarian and adrenal gland tumours, which can lead to an increase in the production of androgens.

Hirsutism is an extremely distressing condition for the women who suffer from it and has a very negative impact on the quality of life and the psychosocial development of patients.

The prevalence of hirsutism may depend on the method used to determine its presence and the population concerned. The typical strategy applied for the diagnosis of this condition consists in an evaluation of hair growth in 11 areas of the body and the assignment of a score of 0 to 4, a method introduced in the 1960s by Ferriman and Gallway (N. Sonino, G. A. Fava, E. Mani, P. Belluardo, and M. Boscaro, "Quality of life of hirsute women", Postgrad. Med. J., vol. 69, n. 809, pp. 186-9, 1993).

The total prevalence of hirsutism is not known, but it is estimated that it ranges from 5 to 10% of women in fertile age. The difficulty in determining the exact incidence and prevalence of this disorder depends on the fact that there exists great variability in the quantity of hair in the population, due to genetic and environmental factors. Some populations, in fact, may exhibit a small quantity of body hair, whereas others (Mediterranean, Middle Eastern and Southeast Asian populations) normally exhibit greater quantities of hair, plasma androgen levels being equal.

The prevalence of hirsutism after menopause is rather high, with about 50% of women reporting an increase in the growth of facial hair.

In most cases hirsutism is a sign of a hormonal disorder, hyperandrogenism, which is characterised by an increase in androgen synthesis. In some cases, however, the increase in hair growth is not associated with an increase in androgens, for example in the case of the "idiopathic hirsutism".

The most common cause of an excess of androgens is polycystic ovary syndrome, but other very important causes include adrenal hyperplasia, characterised by a lack of 21-hydroxylase, acanthosis nigricans syndrome, tumours that produce androgens and the intake of androgens. In any case, there is a difference between an excessive plasma androgen concentration and hirsutism, since the proportionality of the two factors is not always linear.

Hair is essentially composed of keratinised melanocytes, which grow from the dermal papillae within the sheath of the hair follicle. There are approximately 50 million hair follicles which are generally associated with sebaceous glands: 20% of them are situated on the scalp.

The hair growth cycle is characterised by 3 phases:
1. *Anagen*: the active hair growth phase. Hair length depends on the duration of this phase.
2. *Catagen*: the stage in which hair growth stops and the bulb undergoes involution.
3. *Telogen*: in this phase, hair loss is observed.

Hair growth is influenced by different hormones. Growth hormones can lead to generalised hair growth. Thyroid hormones, by contrast, are negatively correlated to hair growth: in the case of a dysfunction of these hormones, hair loss is observed. The hormones estradiol and progesterone have a minimal influence on hair growth. Androgenic hormones are the main regulators of hair growth. Androgens are capable of increasing the rate of growth and transforming the vellus of androgen-sensitive areas into terminal hair.

Testosterone is the main circulating androgenic hormone and is converted to 5α-dihydrotestosterone (DHT) by the enzyme 5α-reductase. Testosterone and DHT are capable of stimulating dermal papillae to form terminal hair. Different areas of the skin can be more or less sensitive to the effect of androgens, probably because of variations in the androgen and 5α-reductase content.

There is a possibility of treating hirsutism by pharmacological means, but the therapies presently available result in side effects that may be particularly marked and they do not have proven effectiveness.

Pharmacological treatments must be limited to the patients that have no intention of carrying to term a pregnancy. There is little experimental evidence on the effectiveness of pharmacological treatments of hirsutism. In most cases, clinical studies are conducted on a limited number of patients and with treatments of brief duration; moreover, clinical studies are often not blind and the assessment of effectiveness is not objective.

The main therapeutic approaches are the following:
1. Topical application of eflornithine.
   Eflornithine is an active ingredient which inhibits the enzyme ornithine decarboxylase, involved in the biosynthesis of putrescine, a factor capable of regulating the growth and differentiation of hair follicle cells. Introduced initially in therapy for the treatment of African trypanosomiasis, it was later approved for the topical treatment of hirsutism, in the form of a 13.9% cream. It is a fairly effective treatment; however, it is not free of problems. In fact, it was approved only for the reduction of facial hair and it is not possible to apply it in other areas of the skin, due to the side effects that could result from an excessive systemic absorption. Moreover, it is a highly costly active ingredient.
2. Oral contraceptives. Oral contraceptives suppress the synthesis of androgens by inhibiting the secretion of gonadotropin by the pituitary gland and the increase in the production of SHBG (sex hormone binding globulin), which reduces the concentration of free testosterone in plasma. Oral contraceptives should be chronically administered but in any case not all of the clinical studies conducted to date have led to consistent results as to their effectiveness. Furthermore, their use can be associated with an increase in arterial pressure and the production of blood coagulation factors, with an increase in cardiovascular risk.
3. Suppression of androgens. The main agents are androgen receptor antagonists, including spironolactone, flutamide and cyproterone acetate and 5α-reductase inhibitors, such as finasteride. All of these drugs are teratogenic and must be used together with a safe, suitable contraceptive.
4. Insulin sensitizers. It has been demonstrated that weight loss or the use of metformin or thiazolidinediones can reduce the production of androgens and improve the condition of women with polycystic ovary syndrome. However, the ability of these therapies to positively influence hirsutism correlated to PCOS still needs to be clarified.

At present, there is no available treatment for hirsutism, or for reducing the rate of hair growth, which is tolerable by the majority of individuals and effective and which is practically free of side effects or has extremely reduced side effects compared to the pharmacological therapies used until now WO 2009/052518 discloses a composition for the reduction of facial hair hirsutism in females comprising a combination of two active agents, one of them being a type 1 5-alpha reductase inhibitor.

The present invention provides a composition according to the claims hereunder.

The present invention relates to a composition comprising at least one excipient and/or pharmaceutically acceptable and/or food grade technological additive and a mixture which comprises, or, alternatively, consists of at least the following components:
(a) at least one isoflavone, preferably genistein, daidzein, glycitein and combinations thereof;
(b) at least one sterol, preferably β-sitosterol, cycloartenol, stigmasterol and combinations thereof; and
(c) apigenin.

The subject matter of the present invention relates to the composition of the invention as indicated above for use in the curative or preventive treatment of hirsutism, wherein said treatment comprises the topical application of said composition.

The subject matter of the present invention further relates to the non-therapeutic use of a composition as described above for reducing the rate of hair growth.

The present invention further relates to a pharmaceutical or cosmetic composition or a medical device comprising the composition as described above.

Unless specified otherwise, the content of a component or substance in a composition refers to the percentage by weight of that component or substance relative to the total weight of the composition.

Unless specified otherwise, the indication that a composition "comprises" one or more components or substances means that other components or substances can be present in addition to the one or ones specifically indicated.

The present invention will also make reference to the following Figures:
Figure 1: photographic documentation for product B at time T0, T1, T3 in volunteer no. 2
Figure 2: photographic documentation for product B at time T0, T1, T3 in volunteer no. 10
Figure 3: diagram of *in vivo* study data related to the rate of hair regrowth for product A (placebo) and product B (product according to the invention) at time T0, T1, T3;
Figure 4: diagram of *in vivo* study data related to vellus hair density for product A (placebo) and product B (product according to the invention) at time T0, T1, T3;
Figure 5: diagram of *in vivo* study data related to terminal hair density for product A (placebo) and product B (product according to the invention) at time T0, T1, T3;
Figure 6: diagram of *in vivo* study data related to total hair density for product A (placebo) and product B (product according to the invention) at time T0, T1, T3;
Figure 7: diagram of *in vivo* study data related to skin surface electrical capacitance (hydration) for product A (placebo) and product B (product according to the invention) at time T0, T1, T3;

Isoflavones are chemical compounds endowed with numerous pharmacological activities, including activity as an oestrogen receptor agonist and anti-inflammatory and antioxidant activities. They are synthesised almost exclusively from plants belonging to the family Fabaceae (also known as Leguminosae), such as *Glycine max*, *Phaseolus vulgaris* L., *Medicago sativa* L., *Vigna radiata* L., *Vigna unguiculata* L., *Pueraria lobata* L. and *Trifolium pratense* L.

*Glycine* is a genus of plants belonging to the family Fabaceae, native of Asia. The subgenus Soja comprises two botanical species, *Glycine soja* and *Glycine max,* widely used in human and animal diets. *Glycine soja sterols* is the INCI term (European nomenclature for the ingredients of a cosmetic product) with which phytosterols obtained from soybean (*Glycine soja, Fabaceae*) are identified.

*Glycine soja seed extract* is the INCI term with which extracts obtained from soybean (*Glycine soja, Fabaceae*) are identified.

The chemical compounds characteristic of soy extract are isoflavones and phytosterols, whose concentration varies according to the part of the plant used for the extract.

The main isoflavones contained in soy are genistein, daidzein and glycitein. The main sterols are β-sitosterol, cycloartenol and stigmasterol. These compounds are endowed with different pharmacological activities: isoflavones act as oestrogen receptor agonists and have shown antiandrogenic activity in vitro and in vivo, whilst phytosterols have an antioestrogenic and antiandrogenic activity. These activities could be useful in limiting the rate of the hair growth that occurs in disorders such as hirsutism.

The hormones mainly involved in the pathogenesis of hirsutism are testosterone and 5α-dihydrotestosterone (DHT).

Testosterone is the most abundant androgen present in plasma, where only 3% is in free form, whilst the rest is bound to albumin and sex hormone binding globulin (SHBG). Testosterone is synthesised in the Leydig cells, situated in the testicles, as a result of the luteinising hormone (LH). The circulating testosterone is internalised in cells, where it is converted into dihydrotestosterone (DHT) as a result of the action of the enzyme 5α-reductase. Dihydrotestosterone is the preferential ligand of the androgen receptor and as a result of its binding with the receptor, the complex moves into the nucleus, where it induces the transcription of several genes, known as androgen receptor-regulated genes (ARRGs).

5α-dihydrotestosterone plays an important role in different pathologies, such as benign prostatic hyperplasia and prostatic carcinoma, but also acne, hirsutism and androgenetic alopecia. DHT has 2 to 5 times more affinity for the androgen receptor than testosterone and has a potency of action that is 10 times greater. This suggests that the two steroidal hormones have a different but complementary effect.

The 5α-reductase family is composed of 3 subfamilies and 5 isoenzymes altogether:
1. 5α-reductase type 1 and 2;
2. 5α-reductase type 3;
3. Protein GPSN2 (glycoprotein synaptic 2) and GPSN2L (glycoprotein synaptic 2-like).

All the enzymes have the ability to catalyse the stereospecific reduction reaction of various chemical compounds with a steroidal structure: the preferential substrates of this type of enzyme are 3-oxo (3-keto) steroids, Δ^{4,5}, C 19 (testosterone) and C 21 (progesterone).

Differences exist in the tissue distribution of the enzymes belonging to the 5α-reductase family, which change with age. From puberty up to about 81 years of age, the isoform 1 of the enzyme is prevalently expressed in the skin and scalp. During adult life, the 3 isoforms (5αR1-3) are ubiquitously expressed, with some differences in expression in the liver, skin, prostate, epididymis, testicle, and ovary tissues and other tissues. At the epidermal level, the expression of isoform 1 is found only in the sebaceous glands, but not in the hair follicles or other areas of the epidermis, whereas isoform 2 is present mainly in the hair follicle, but is not found in the dermal papillae or sebaceous glands.

Soy isoflavones have the ability to inhibit different enzymes involved in the synthesis of androgens. In particular, the ability of these chemical compounds to inhibit the enzymes 3β-hydroxysteroid dehydrogenase (3β-HSD), 17β-hydroxysteroid dehydrogenase (17β-HSD) and 5α-reductase has been demonstrated. The inhibition of the latter enzyme leads to a reduction in the levels of DHT, with a consequent reduction in the symptoms of hirsutism.

In a preferred embodiment, in the composition according to the invention the at least one isoflavone (a) is extracted from a plant belonging to the family *Fabaceae*, preferably belonging to one of the genera *Glycine*, *Phaseolus*, *Medicago*, *Vigna*, *Pueraria* and/or *Trifolium,* more preferably belonging to the species *Glycine max* or *Glycine soja.* Said isoflavone (a) is preferably extracted from sprouts or seeds or oil extracted from at least one of said plants.

In the composition according to the invention, the isoflavone (a) is preferably genistein, daidzein, glycitein and combinations thereof.

Phytosterol is a term which comprises sterols and stanols of plant origin, two classes of chemical compounds characterised by a structure similar to that of cholesterol. Phytosterols can be extracted from vegetable oils such as maize oil (*Zea mays* L.), rapeseed oil (*Brassica napus* L.), soybean oil (*Glycine max* (L.) Merr.), sunflower oil (*Helianthus annuus* L.) or olive oil (*Olea europaea* L.). They are also present to a lesser degree in nuts, seeds fruit and vegetables. The most abundant sterols are sitosterol, campesterol and stigmasterol: they constitute approximately 65%, 30% and 3%, respectively, of the consumption of plant sterols in the diet.

Phytosterols are the main chemical compounds contained in soybean oil. There are variations in the qualitative and quantitative composition of the phytosterols present in different parts of *Glycine max* (L.) Merr. In particular, sitosterol is the main sterolic compound of sprouts, whereas a larger quantity of cycloartenol is present in seeds.

β-sitosterol is one of the most abundant phytosterols present in the plant kingdom. One of the most important activities consists in the inhibition of the enzyme 5α-reductase. In a study conducted on rats subjected to a gonadectomy, an increase in prostate volume was observed following transdermal administration of testosterone. The co-administration of sitosterol and testosterone, by contrast, reduced this phenomenon in a dose-dependent manner. In the same study two in vitro tests were conducted in order to determine whether the inhibition of the increase in prostate volume was induced by the inhibition of the enzyme 5α-reductase or by an antagonist effect on the androgen receptor. In the study, a good inhibitory activity against 5α-reductase was observed, with an IC₅₀ of 2.7 µM. The same study demonstrated that sitosterol has a low affinity for the androgen receptor: this demonstrates that the anti-androgenic activity depends on the inhibition of DHT synthesis.

In the compositions according to the invention, the sterol (b) can be of natural origin (phytosterols) or synthetic origin.

Non-limiting examples of sterols (b) of natural origin are sterols obtained from a soybean extract (of seeds, sprouts or other parts of soybean), for example extracts of *Glycine soja* or *Glycine max* or combinations thereof

Non-limiting examples of sterols (b) of natural origin are β-sitosterol, cycloartenol, stigmasterol.

In the composition according to the invention, the sterol (b) is preferably β-sitosterol, cycloartenol and/or stigmasterol and combinations thereof.

Apigenin (4',5,7-trihydroxyflavone) is a chemical compound belonging to the class of the flavonoids, specifically to the subclass of the flavones, very widespread in nature, where it can be found in an aglycone form and in the form of a glycoside, such as rhoifolin (or roifolina) and isovitexin.

This compound is found in abundance in various plant species, including chamomile (*Matricaria recutita* L., *Matricaria chamomilla* L., *Matricaria discoidea* L., *Chamaemelum nobile* (L.) All.), parsley (*Petroselinum crispum*), celery (*Apium graveolens* L., *Apium graveolens* L. var. *rapaceum*), tea (*Camellia sinensis* L.) and grapefruit (*Citrus x paradisi* Macfad.).

In the composition according to the present invention, the apigenin (c) is preferably extracted from at least one plant of the genera *Matricaria, Chamaemelum, Apium, Petroselinum, Camellia* and/or *Citrus.*

Apigenin exhibits various pharmacological activities and one of the most interesting for the purposes of the present invention is its ability to inhibit the enzyme ornithine decarboxylase.

Polyamines have long been known as important cell growth factors, though their role has not yet been completely clarified. The concentrations of polyamines are strictly regulated by the synthesis, transport and degradation thereof. Their synthesis is mainly regulated by the enzymes ornithine decarboxylase (ODC1) and adenosylmethionine decarboxylase, where the degradation is regulated by the enzyme spermidine/spermine N1-acetyltransferase (SSAT).

Ornithine decarboxylase is the enzyme that catalyses the step limiting the synthesis of polyamines and has shown its importance in the development of different tissues, such as those of the brain, teeth, testicles and pancreas.

Its action is crucial also in hair growth, as demonstrated in 1983 by Takigawa, who demonstrated that the inhibition of this enzyme by α-difluoromethylornithine leads to the inhibition of hair growth. Numerous other studies have demonstrated a correlation between the inhibition of this enzyme by difluoromethylornithine and a marked reduction in hair growth. In one study, this compound led to the onset of alopecia and dermatitis in rats and dogs. In other studies it was demonstrated that overexpression of this enzyme in hair follicles is associated with hair loss, excessive skin folds and tumour formation. These studies demonstrate that hair follicles can be sensitive to changes in the concentration of this enzyme.

The levels of ornithine decarboxylase increase during the *anagen* phase of hair growth and are drastically reduced during the *catagen* phase. The up-regulation of SSAT or ODC1 can lead to hair loss and cutaneous cysts in rats.

One in vivo study demonstrated the effectiveness of the topical application of a solution of apigenin in the inhibition of epidermal ornithine decarboxylase in mice. The study provided for the application, on the mouse's skin, in an area of 4 cm², of 0.2 mL of a solution of apigenin in DMSO at different concentrations; the cutaneous application of the solution of apigenin was followed by the application of 17 nmol of 12-0-tetradecanoylphorbol-13-acetate (TPA) in 0.2 mL of acetone to induce the expression and activity of ornithine decarboxylase. Six hours after the application of TPA, the mice were sacrificed and the activity of ornithine decarboxylase was calculated. The maximum percentage of inhibition of this enzyme was found at the concentration of 20 µmol of apigenin in 0.2 mL.

After extended research activity it was found that the active ingredients (a), (b) and (c) of the composition according to the present invention have a synergistic effect.

The present invention makes it possible to obtain, simultaneously:
- An anti-androgenic effect;
- Reduction of the proliferation of hair follicle cells.

The final effect of the composition of the invention consists in a reduction in the rate of body hair growth, as well as a reduction in the shaft diameter and hair pigmentation.

The composition of the invention further reduces the total hair density and in particular the density of terminal hair (hair with a diameter >0.04 mm), i.e. hair in the active phase of regrowth, thus generating conditions of thinner, sparser hair.

Additionally, the composition of the invention is well tolerated and does not have any relevant side effects. The composition of the invention leaves the skin surface smooth and hydrated.

When used for cosmetic purposes, the composition of the invention shows to have good cosmetic acceptability in terms of spreadability, texture, absence of residues after its application on skin, scent and colour.

Finally, the composition of the invention is economical and easy to produce.

Isoflavones and sterols are capable of inhibiting various enzymes involved in the synthesis of steroidal hormones: 3β-hydroxysteroid dehydrogenase (3β-HSD), 17β-hydroxysteroid dehydrogenase (17β-HSD) and 5α-reductase. In particular, the reduction of the latter enzyme enables the concentration of dihydrotestosterone in the hair follicle to be reduced, resulting in a reduction in androgen receptor activation and a consequent lower rate of hair growth.

By acting on the enzyme ornithine decarboxylase, apigenin can lead to a reduction in the concentration of putrescine, an important growth factor that regulates the proliferation of hair follicle cells. The lower concentration of putrescine should slow down the proliferation of follicle cells, with a consequent reduction in the rate of hair growth.

The synergistic effect of the composition of the present invention derives from a fact that the pharmacologically active compounds interact on different molecular targets.

In the present invention, the synergistic action takes place between isoflavones, sterols and apigenin. The synergy occurs in an optimal manner when the isoflavones, preferably extracted from a plant belonging to the family Fabaceae, preferably from seeds, sprouts or oil extracted from *Glycine max* or *Glycine soja,* are present at a concentration comprised from 0.05% to 10%, the sterols, of natural or synthetic origin, are present at a concentration comprised from 0.1% to 20 % and the apigenin, preferably extracted from at least one plant of the genus *Matricaria*, *Chamaemelum, Apium, Petroselinum, Camellia* and/or Citrus is present at a concentration comprised from 0.1% to 20%.

In the composition according to the present invention, (a) is preferably present in an amount of 0.05% to 10%, more preferably 0.1 to 5%, even more preferably from 0.5 to 2%, and/or (b) is present in an amount of 0.1 to 10%, more preferably 0.5 to 5%, even more preferably 1 to 3%, and/or (c) is present in an amount of 0.1 to 10%, more preferably 0.5 to 10%, even more preferably 0.5 to 3%, wherein all the amounts refer to the weight of the component relative to the total weight of the composition.

The composition according to the present invention is preferably in the form of a cream, ointment, gel, solution, suspension, emulsion, powder, spray, gauze, patch or other forms of delivery suitable for topical use.

The composition according to the present invention preferably comprises at least the following components:
a)

| *Active ingredient* | *Concentration by weight*/*total weight* |
|---|---|
| Sterol or mixture of sterols | 2-3% (preferably 2.5%) |
| Apigenin | 1.5-2.5% (preferably 2%) |
| Isoflavones | 0.25-0.75% (preferably 0.5%) |

Or, alternatively,
b)

| *Active ingredient* | *Daily dose* |
|---|---|
| Sterol or mixture of sterols | 2-3% (preferably 2.5%) |
| Apigenin | 1-2% (preferably 1.5%) |
| Isoflavones | 0.25-0.75% (preferably 0.5%) |

c)

| *Active ingredient* | *Daily dose* |
|---|---|
| Sterol or mixture of sterols | 2-3% (preferably 2.5%) |
| Apigenin | 0.1-1% (preferably 0.5-0.8%) |
| Isoflavones | 0.25-0.75% (preferably 0.6%) |

Pharmaceutically acceptable and/or cosmetic grade and/or food grade excipient and/or technological additive means any substance, or combination of substances, auxiliary to the production of a pharmaceutical, cosmetic, food or nutraceutic form found in the finished product that is not the active ingredient, even if it can modify the stability, release or other characteristics thereof.

Non-limiting examples of such ingredients, as known to the person skilled in the art of formulations in the pharmaceutical, nutraceutic or food sectors, are diluent, absorbent, adsorbent, lubricant, glidant, colouring, surfactant, antioxidant, sweetening, flavouring, binding, disintegrating, plasticising, viscosity enhancing, emulsifying, humectant, wetting, preservative and chelating excipients and the like.

In one embodiment, the present invention relates to a pharmaceutical or cosmetic composition or a medical device comprising the composition as described above.

In the context of the present invention, the term "medical device" is used with the meaning according to Italian Legislative Decree no. 46 of 24 February 1997, i.e. it indicates a substance or another product, whether used alone or in combination, intended by the manufacturer to be used for human beings for the purpose of diagnosis, prevention, monitoring, treatment or alleviation of a disease, and which does not achieve its principal intended action in or on the human body for which it is intended by pharmacological, immunological or metabolic means, but which may be assisted in its function by such means.

The composition of the present invention is to be understood as for either human or veterinary use, i.e. as a preparation to be applied to animals with the uses and methods known to the person skilled in the art.

In the context of the present invention, "treatment" means an intervention comprising the administration of a substance, or mixture of substances, having the aim of eliminating, reducing or preventing a pathology and the symptoms thereof.

The following examples are provided in order to illustrate some embodiments of the invention, without any intention of limiting the scope thereof.

### Composition 1:

| INGREDIENTS (INCI nomenclature) | CONCENTRATION |
|---|---|
| AQUA | 65.00-75.00% |
| DIETHYLEXYL ADIPATE | 1.00-5.00% |
| GLYCERIN | 1.00-5.00% |
| ISOHEXADECANE | 1.00-5.00% |
| DICAPRYLYL CARBONATE | 1.50-3.50% |
| GLYCINE SOJA STEROLS | 1.50-3.50% |
| PROPYLENE GLYCOL | 1.50-3.50% |
| CYCLOPENTASILOXANE | 1.00-3.00% |
| APIGENIN | 1.00-2.00% |
| CETYL ALCOHOL | 0.78-1.78% |
| GLYCERYL STEARATE | 0.77-1.77% |
| PHENOXYETHANOL | 0.10-1.90% |
| SOYBEAN GLYCERIDES | 0.10-1.90% |
| GLYCINE SOJA SEED EXTRACT | 0.10-1.60% |
| BISABOLOL | 0.05-1.00% |
| DIMETHICONE | 0.05-1.00% |
| TOCOPHEROL | 0.05-1.00% |
| PEG-75 STEARATE | 0.05-1.00% |
| CARBOMER | 0.05-1.00% |
| PARFUM (FRAGRANCE) | 0.05-1.00% |
| BUTYROSPERMUM PARKII BUTTER UNSAPONIFIABLES | 0.01-0.60% |
| TOCOPHERYL ACETATE | 0.01-0.60% |
| CETETH-20 | 0.01-0.50% |
| STEARETH-20 | 0.01-0.50% |
| SODIUM HYDROXIDE | 0.01-0.50% |
| ETHYLHEXYLGLYCERIN | 0.01-0.50% |
| ALOE BARBADENSIS LEAF JUICE | 0.01-0.50% |
| PPG-15 STEARYL ETHER | 0.01-0.50% |
| TETRASODIUM EDTA | 0.001-0.500% |
| GLYCERYL OLEATE | 0.001-0.500% |
| BHT | 0.001-0.500% |
| HEXYLENE GLYCOL | 0.001-0.500% |
| BHA | 0.001-0.500% |
| PSEUDOALTEROMONAS EXOPOL YSACCHARIDES | 0.001-0.500% |
| CITRIC ACID | 0.001-0.500% |
| SODIUM SALICYLATE | 0.001-0.500% |
| ASCORBYL PALMITATE | 0.00001-0.05000% |
| POTASSIUM SORBATE | 0.00001-0.05000% |
| SODIUM BENZOATE | 0.00001-0.05000% |
| SODIUM SULPHITE | 0.00001-0.05000% |

### Composition 2

| INGREDIENTS (INCI nomenclature) | CONCENTRATION |
|---|---|
| AQUA | 90.684129% |
| GLYCINE SOJA STEROLS | 2.500000% |
| PROPYLENE GLYCOL | 2.275000% |
| APIGENIN | 0.807500% |
| PEG-40 HYDROGENATED CASTOR OIL | 0.800000% |
| GLYCINE SOJA SEED EXTRACT | 0.612500% |
| ALOE BARBADENSIS LEAF JUICE | 0.518232% |
| PRUNUS AMYGDALUS DULCIS OIL | 0.500000% |
| TOCOPHEROL | 0.500000% |
| SODIUM BENZOATE | 0.300520% |
| PARFUM (FRAGRANCE) | 0.200000% |
| POTASSIUM SORBATE | 0.150520% |
| TETRASODIUM EDTA | 0.100000% |
| LACTIC ACID | 0.050000% |
| CITRIC ACID | 0.001100% |
| SODIUM SULPHITE | 0.000440% |

### Composition 3

| INGREDIENTS (INCI nomenclature) | CONCENTRATION |
|---|---|
| AQUA | 75.00-95.00% |
| GLYCERIN | 1.00-5.00% |
| GLYCINE SOJA STEROLS | 1.00-5.00% |
| PROPYLENE GLYCOL | 1.00-5.00% |
| STEARETH-20 | 1.00-5.00% |
| ALOE BARBADENSIS LEAF JUICE | 0.50-3.00% |
| GLYCINE SOJA SEED EXTRACT | 0.10-2.00% |
| APIGENIN | 0.10-2.00% |
| SODIUM BENZOATE | 0.10-2.00% |
| GLYCERYL LAURATE | 0.10-2.00% |
| BISABOLOL | 0.10-2.00% |
| POTASSIUM SORBATE | 0.01-1.00% |
| DISODIUM EDTA | 0.01-1.00% |
| PARFUM (FRAGRANCE) | 0.01-1.00% |
| MORINGA OLEIFERA SEED EXTRACT | 0.01-1.00% |
| CITRIC ACID | 0.0001-0.0100% |
| SODIUM SULPHITE | 0.0001-0.0100% |

### EXPERIMENTAL PART

Studies conducted on the composition of the present invention for reducing the rate of hair growth and for the prevention and/or treatment of hirsutism.

The effect of a composition of the present invention and of the individual constituents in reducing the rate of hair growth was evaluated (in order to observe the synergistic effect among the individual components).

### 1. RATE OF HAIR REGROWTH: in vivo study in animals

In order to be able to evaluate the synergistic effect of the composition, the rate of hair growth in a mouse model, as previously described, was evaluated (J. Y. Lee, K. R. Im, T. K. Jung, M.-H. Lee, and K.-S. Yoon, "Medicinal Herbal Complex Extract with Potential for Hair Growth-Promoting Activity", J. Soc. Cosmet. Sci. Korea, vol. 38, n. 4, pp. 277-287, 2012; K. Shin, T. Kim, J. Kyung, D. Kim, D. Park, E. Choi, "Effectiveness of the combinational treatment of Laminaria japonica and Cistanche tubulosa extracts in hair growth", Lab Anim Res, vol. 31, n. 1, pp. 24-32, 2015).

For the study, use was made of C57BL/6 mice, which were housed in temperature-controlled rooms, with a temperature of 23 ± 2 °C, relative humidity of 45-65% and with light/dark cycles of 12 hours. The animals had free access to water and food, consisting in a standard diet. All experiments were performed in observance of Legislative Decree no. 116 of 27 January 1992 and according to the guidelines of the Council of the European Union (86/609/EEC and 2010/63/EU).

The *anagen* phase of the hair present on the back of the mice was induced by following one of the procedures described in the literature (S. Muller-Rover, B. Handjiski, C. Van Der Veen, S. Eichmüller, K. Foitzik, I. A. McKay, K. S. Stenn, and R. Paus, "A comprehensive guide for the accurate classification of murine hair follicles in distinct hair cycle stages", Journal of Investigative Dermatology, vol. 117, no. 1. pp. 3-15, July 2001.). In short, after having adapted the mice to the new environment for a week, the *anagen* phase was induced by shaving the hair on the back, so as to synchronise the *anagen* phase in all animals. Starting from the day after shaving, the formulations were applied on the skin of each animal, 2 times a day for 28 days, using a specific vehicle. The promotion of hair growth was evaluated by means of photographs of the rats and histological examination.

For an objective comparison of the rate of hair regrowth, photographs were taken of the back of the rats and the percentage area of hair growth was calculated by means of specific software.

One mouse taken from each group was sacrificed after 14, 21 and 28 days from the start of the treatment. The skin of the back was cut into a section with a diameter of 5 µm, fixed in formalin, embedded in paraffin and stained with hematoxylin/eosin for the histological examination.

### 2. EVALUTATION OF THE HAIR REGROWTH SLOWING EFFECTIVENESS: in vivo study in humans

### A. AIM OF THE STUDY

The hair regrowth slowing effectiveness of a cosmetic after epilation according to the invention was clinically evaluated using non-invasive instrumental investigations (product B corresponding to the above-described composition 2), applied twice a day (morning and evening) on the legs for a continuous period of 3 months, by informed and consenting female volunteers, with hypertrichosis of the legs; the study involved an evaluation of the activity of the product under examination compared to a "placebo" formulation (product A) (within-subject comparison).

Furthermore, an assessment was made of the degree of cosmetic acceptability of the product under examination, as expressed by the volunteers, as well as the opinion expressed by the subjects at the end of the trial as to the effectiveness and tolerability of the same.

### B. METHOD

Double-blind, randomised, placebo-controlled clinical study, which provided for the use of the products studied twice a day, morning and evening, for a continuous period of 3 months.

In particular, each volunteer who met the inclusion criteria performed a topical treatment with the product studied (product B) and with the placebo (product A) on the legs (one product on the right leg and the other on the left leg on the basis of a previously defined randomisation list). The assigned side for each product remained the same throughout the duration of the study.

Three examinations were carried out in the course of the study: a baseline examination (T0), an intermediate examination (T1: after one month of treatment) and a final examination at the end of the trial (T3: after 3 months of treatment).

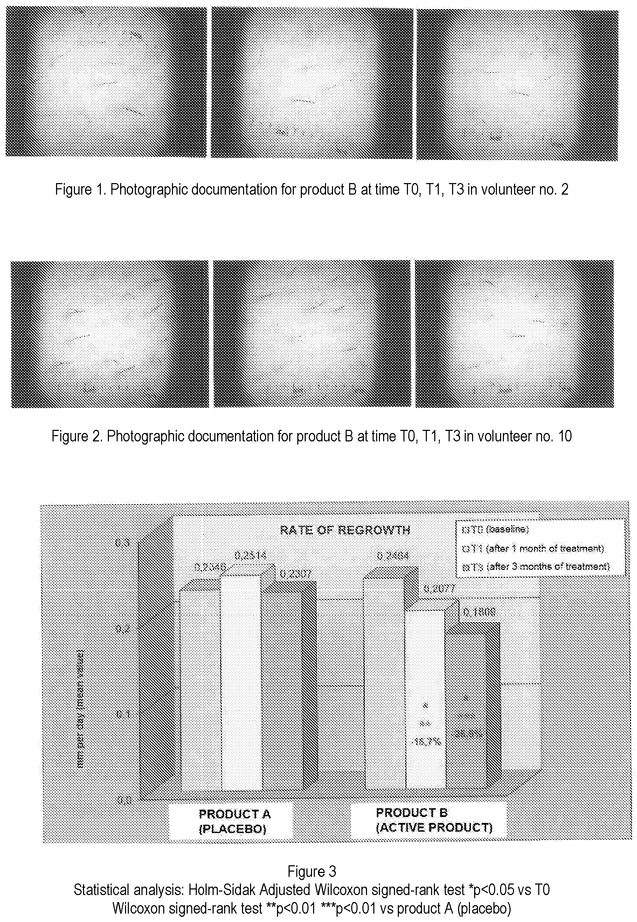

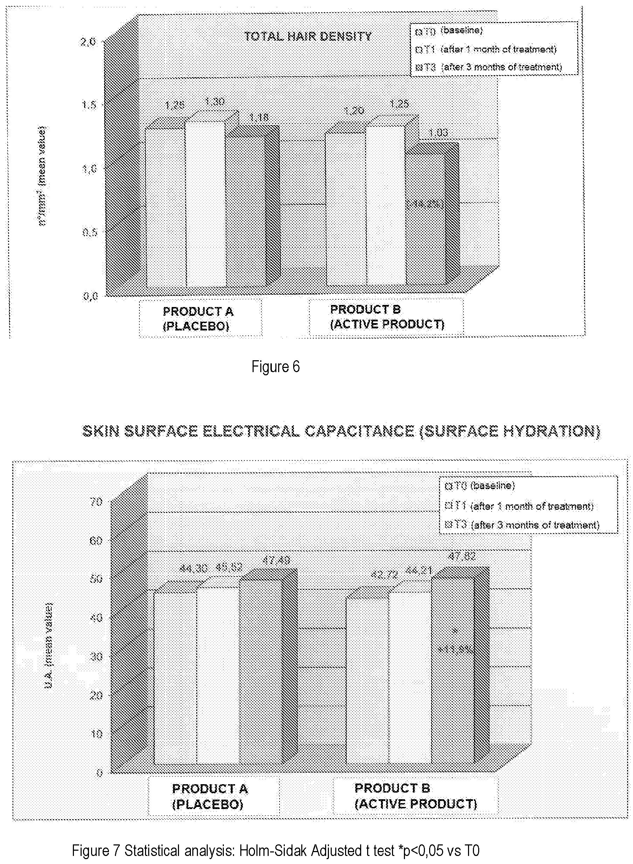

An evaluation of the activity of the product according to the invention (B) /placebo (A) was carried out by means of:
- A phototrichogram (Figure 1, 2), an image analysis for determining the parameters provided for by the experimental protocol (rate of hair regrowth, total hair density, terminal hair and vellus hair density) (Figure 3-6).
- Measuring the skin surface electrical capacitance (hydration) by means of a Corneometer CM825 (Courage - Khazaka, Köln Germany) (Figure 7).

At the end of the study, the volunteers provided a subjective assessment of effectiveness and tolerability of the tested products; furthermore, the experimenter considered their adherence to the treatment and any events that could have interfered with/influenced the result of the trial.

The collection of digitised images for the phototrichogram was carried out for each experimental period (T0, T1 and T3) 7 days after epilation with pull-off strips (cold or hot waxing) using a digital video dermatoscope; the site selected for the study was a defined, very localised area of the tibial region, about 1 cm² in size, whose identification was ensured, for each of the aforesaid periods, by using a special mask adapted to the leg of each volunteer.

### C. STUDY POPULATION

The test was carried out on 24 informed and consenting female volunteers, of an age comprised between 21 and 56 years (mean age = 38), with hypertrichosis of the legs, who habitually use pull-off strips as their hair removal system.

### D. RESULTS

All the results are reported in detail in tables 1-2 and in figures 1-7. The data were processed according to descriptive and inferential statistical methods.

No important events were observed which may have influenced the results of the tests.

Two subjects dropped out during the test; in fact, volunteers 15 and 20 interrupted the study prematurely because of personal problems. The statistical analysis was carried out on data relating to the 22 subjects who completed the study as per procedure.

The activity of the product studied was expressed in absolute values versus the baseline value and in comparison to the placebo; the following were taken into consideration in particular:
- the comparison between the different evaluation periods versus the baseline (T1 and T3 vs T0) for each of the products studied (product A and B) by means of parametric tests (ANOVA with repeated measurements), when the normality hypothesis was confirmed by the Shapiro-Wilks normality test (threshold value of 5%) or non-parametric tests (Friedman test) when the normality hypothesis was rejected, followed, in the event of a statistically significant result, by the Holm-Sidak test.
- the comparison between the two tested products (product A and B) by means of Student's t test for paired data (paired t test) in the event in which the distribution of data was normal (p<0.05 Shapiro-Wilk test); otherwise (p<0.05 Shapiro-Wilk test) the data analysis was carried out by means of the Wilcoxon test.

### D.1. Evaluation of effectiveness

### D.1.1. Phototrichogram

The results obtained in relation to the product of the invention (product B) showed, already at T1 and more markedly at T3, a statistically and clinically significant reduction (Holm-Sidak Wilcoxon signed-rank test T1 and T3 vs T0) in the hair regrowth rate versus the baseline corresponding to 15.7 % and 26.6%, respectively. These reductions were statistically different compared to the placebo (Wilcoxon signed-rank test p<0.01 T1_{product B} vs T1_{product A}; p<0.001 T3_{product B} vs T3_{product A}); moreover, in the case of the placebo, no significant variations were observed in the parameter considered versus T0 (Figure 3).

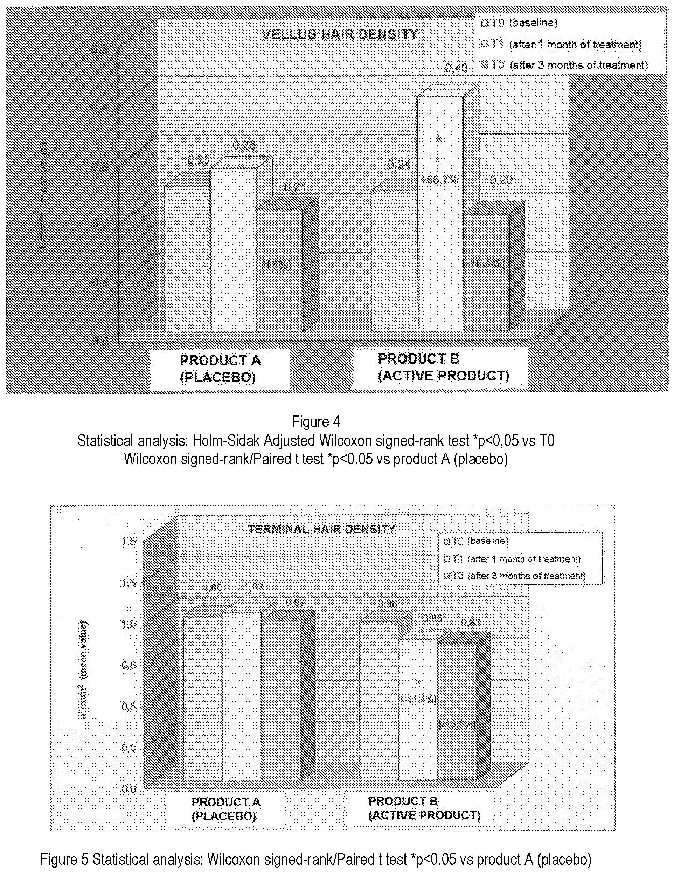

With regard to the other parameters considered (total hair density, terminal hair and vellus hair density) in relation to the product of the invention (product B), it is important to note a significant increase at T1 (+66.7%) (Holm-Sidak Wilcoxon signed-rank test T1 vs TO) in vellus hair (hair with a diameter of 0.04 mm) density (Figure 4) and a trend toward a reduction (-11.4%) in terminal hair (hair with a diameter > 0.04 mm) (Figure 5), indicative of an initial process of "miniaturisation" thereof statistically different compared to the placebo (vellus hair and terminal hair, Wilcoxon signed-rank/Paired t test p<0.05 T1_{product B} vs T1_{product A}).

At T3, this process of "weakening" the terminal hair translates into a more marked reduction in the density thereof (-13.5%) and in a clinically significant reduction in total hair density (-14.2%) (Figure 5); at the end of the period of application the terminal hairs were thus less numerous and sparser.

In the case of the placebo (product A), at T3 no significant variations were observed in the terminal hair density (-3.0%) or total hair density (-5.6%) versus T0.

The observed trend toward a reduction in vellus hair by the product of the invention (product B, -16.7%) at T3, wholly comparable to the reduction obtained for the placebo (product A, -16,0%), confirms that hair in the active regrowth phase is most susceptible to the treatment in study.

### D.1.2. Skin surface electrical capacitance (hydration)

After 3 months of treatment (T3), a statistically significant increase in skin surface electrical capacitance (hydration) was observed for the product according to the invention (product B), equal to +11.9% versus the baseline (Holm-Sidak Adjusted t test p<0.05 T3 vs T0) (Figure 7).

Though no significant differences were observed between the two treatments compared, it is important to note that the application of the placebo (product A) did not bring about any clinically/statistically significant variation in the parameter considered.

### D. 1.3. Opinion of the subjects

### D. 1.3.1. Opinion on effectiveness

The effectiveness of the product according to the invention (product B) on the hair regrowth rate after depilation was noted by 91% of the subjects; furthermore, at the end of the study, 91% of the subjects declared that they had finer hair and 81.8% that they had sparser hair (decrease in the number of hairs) (Table 1).

The effectiveness of the placebo (product A) on the same parameters was less (73% for the rate of regrowth, 73% for finer hair and 68% for sparser hair) (Table 2) than that of the product according to the invention (product B).

With regard to skin smoothness and hydration, the percentages obtained for the two products A and B are very similar (Tables 1 and 2).

**Table 1 Self-assessment questionnaire on final effectiveness for product B (product of the invention)**

| | **% SUBJECTS** | | | | |
|---|---|---|---|---|---|
| | **absent** | **slight** | **medium** | **marked** | **very marked** |
| **Decrease in the rate of hair regrowth** | 9% | 50% | 14% | 27% | 0% |
| **Decrease in hair thickness** | 9% | 41% | 32% | 18% | 0% |
| **Decrease in the number of hairs** | 18,2% | 36,4% | 36,4 % | 9% | 0% |
| **Improvement in skin hydration** | 5% | 27% | 36% | 23% | 9% |
| **Improvement in skin smoothness** | 4,5% | 1 8% | 41% | 32% | 4,5% |
| **Overall effectiveness of the treatment** | 0% | 14% | 41% | 45% | 0% |

**Table 2 Self-assessment questionnaire on final effectiveness for product A (placebo)**

| | **% SUBJECTS** | | | | |
|---|---|---|---|---|---|
| | **absent** | **slight** | **medium** | I **marked** | **very marked** |
| **Decrease in the rate of hair regrowth** | 27% | 32% | 27% | | 0% |
| **Decrease in hair thickness** | 27% | 41% | 32% | 0% | 0% |
| **Decrease in the number of hairs** | 32% | 36% | 32% | 0% | 0% |
| **Improvement in skin hydration** | 9% | 14% | 46% | 32% | 0% |
| **improvement in skin smoothness** | 9% | 14% | 54% | 23% | 0% |
| **Overall effectiveness of the treatment** | 5% | 32% | 54% | 9% | 0% |

### D. 1.3.2. Cosmetic acceptability

The cosmetic acceptability of both tested products A and B generally showed to be good. In particular, a higher percentage of subjects appreciated the colour (100% of the subjects), scent (100% of the subjects prior to application and 96% after application), texture (100% of the subjects), spreadability (100% of the subjects) and absorption (100% of the subjects) of product B according to the invention, as well as the absence of residues on skin after its application (100% of the subjects).

### D.2. Tolerability

Both products A and B were generally well tolerated; in fact, no adverse events correlated with the formulations under examination were observed. Only subject no. 2 reported occasional itching of a mild entity after the application of both products, lasting a few seconds. However, no significant clinical signs attributable to use of the products under examination were observed; this reaction thus represents a phenomenon to be expected for the type of products tested and is ascribable to a "nonspecific skin reactivity" in a subject with no known allergies and/or intolerances to cosmetics.

The excellent tolerability was moreover confirmed by the opinion of the volunteers: 36% excellent, 59% good insofar as the product according to the invention (product B) was concerned; 45% good, 50% excellent as regards the placebo (product A).

### E. CONCLUSIONS

The results of this study showed that product B according to the invention, already after one month of application, brought about a statistically and clinically significant reduction in the rate of hair regrowth after epilation. At the end of the 3 months of application, the hairs in an active phase of regrowth appeared less numerous and sparser. The study product furthermore demonstrated to possess a good hydrating activity.

The effectiveness of the product was also confirmed by the majority of the subjects who took part in the trial.

No adverse reactions correlatable to use of the treatment were reported.

## Claims

1. A composition comprising at least one pharmaceutically acceptable and/or cosmetic grade and/or food grade excipient and/or technological additive and a mixture which comprises or, alternatively, consists of at least the following components:
(a) at least one isoflavone;
(b) at least one sterol; and
(c) apigenin.

2. The composition according to claim 1, wherein the at least one isoflavone (a) is extracted from at least a plant belonging to the family of the Fabaceae, preferably belonging to one of the genera *Glycine*, *Phaseolus*, *Medicago*, *Vigna*, *Pueraria* and/or *Trifolium,* more preferably belonging to the species *Glycine max* or *Glycine soja.*

3. The composition according to claim 1 or 2, wherein the isoflavone (a) is genistein, daidzein, glycitein and combinations thereof, and/or the sterol (b) is β-sitosterol, cycloartenol and/or stigmasterol and combinations thereof.

4. The composition according to claims 1, 2 or 3, wherein the apigenin (c) is extracted from at least one plant of the genera *Matricaria, Chamaemelum, Apium, Petroselinum, Camellia* and/or *Citrus.*

5. The composition according to one of the preceding claims, wherein (a) is present in an amount of 0.05% to 10% and/or (b) is present in an amount of 0.1 to 10% and/or (c) is present in an amount of 0.1 to 10%, wherein all the amounts refer to the weight of the component relative to the total weight of the composition.

6. The composition according to one of the preceding claims, in the form of a cream, ointment, gel, solution, suspension, emulsion, powder, spray, gauze, patch or other forms of delivery suitable for topical use.

7. The composition according to claims 1-6, comprising the following components:
a)
| *Active ingredient* | *Concentration by weight*/*total weight* |
|---|---|
| Sterol or mixture of sterols | 2-3% |
| Apigenin | 1.5-2.5% |
| Isoflavones | 0.25-0.75% |
or, alternatively,
b)
| *Active ingredient* | *Daily dose* |
|---|---|
| Sterol or mixture of sterols | 2-3% (preferably 2.5%) |
| Apigenin | 1-2% (preferably 1.5%) |
| Isoflavones | 0.25-0.75% (preferably 0.5%) |
or, alternatively,
c)
| *Active ingredient* | *Daily dose* |
|---|---|
| Sterol or mixture of sterols | 2-3% (preferably 2.5%) |
| Apigenin | 0.1-1% (preferably 0.5-0.8%) |
| Isoflavones | 0.25-0.75% (preferably 0.5-0.6%) |

8. A non-therapeutic use of the composition according to one of the preceding claims to reduce hair growth.

9. The composition according to one of claims 1-7, for use in the prevention and/or treatment of hirsutism, wherein said use comprises the topical application of said composition.

10. A pharmaceutical or cosmetic composition or a medical device comprising the composition according to one of claims 1-7.

## Patentansprüche

1. Zusammensetzung, umfassend mindestens einen pharmazeutisch annehmbaren Hilfsstoff und/oder einen Hilfsstoff von kosmetischer Qualität und/oder von Lebensmittelqualität und/oder einen technologischen Zusatzstoff und ein Gemisch, das mindestens die folgenden Komponenten umfasst oder alternativ daraus besteht:
(a) mindestens ein Isoflavon;
(b) mindestens ein Sterol; und
(c) Apigenin.

2. Zusammensetzung gemäß Anspruch 1, wobei das mindestens eine Isoflavon (a) aus mindestens einer Pflanze der Familie der Fabaceae, bevorzugt aus einer der Gattungen *Glycine, Phaseolus, Medicago, Vigna, Pueraria* und/oder *Trifolium,* stärker bevorzugt aus der Art *Glycine max* oder *Glycine soja,* extrahiert ist.

3. Zusammensetzung gemäß Anspruch 1 oder 2, wobei das Isoflavon (a) Genistein, Daidzein, Glycitein und Kombinationen davon ist, und/oder das Sterol (b) β-Sitosterol, Cycloartenol und/oder Stigmasterol und Kombinationen davon ist.

4. Zusammensetzung gemäß Anspruch 1, 2 oder 3, wobei das Apigenin (c) aus mindestens einer Pflanze der Gattung *Matricaria, Chamaemelum, Apium, Petroselinum, Camellia* und/oder *Citrus* extrahiert ist.

5. Zusammensetzung gemäß einem der vorhergehenden Ansprüche, wobei (a) in einer Menge von 0,05% bis 10% vorhanden ist und/oder (b) in einer Menge von 0,1 bis 10% vorhanden ist und/oder (c) in einer Menge von 0,1 bis 10% vorhanden ist, wobei sich alle Mengen auf das Gewicht der Komponente bezogen auf das Gesamtgewicht der Zusammensetzung beziehen.

6. Zusammensetzung gemäß einem der vorhergehenden Ansprüche, in der Form einer Creme, einer Salbe, eines Gels, einer Lösung, einer Suspension, einer Emulsion, eines Pulvers, eines Sprays, einer Gaze, eines Pflasters oder anderen für die topische Anwendung geeigneten Darreichungsformen.

7. Zusammensetzung gemäß einem der Ansprüche 1-6, umfassend die folgenden Komponenten:
a)
| Wirkstoff | Konzentration nach Gewicht/Gesamtgewicht |
|---|---|
| Sterol oder Sterolgemisch | 2-3% |
| Apigenin | 1,5-2,5% |
| Isoflavone | 0,25-0,75% |
oder alternativ,
b)
| | |
|---|---|
| Wirkstoff | Tagesdosis |
| Sterol oder Sterolgemisch | 2-3% (bevorzugt 2,5%) |
| Apigenin | 1-2% (bevorzugt 1,5%) |
| Isoflavone | 0,25-0,75% (bevorzugt 0,5%) |
oder alternativ,
c)
| Wirkstoff | Tagesdosis |
|---|---|
| Sterol oder Sterolgemisch | 2-3% (bevorzugt 2,5%) |
| Apigenin | 0,1-1% (bevorzugt 0,5-0,8%) |
| Isoflavone | 0,25-0,75% (bevorzugt 0,5-0,6%) |

8. Nicht-therapeutische Verwendung der Zusammensetzung gemäß einem der vorhergehenden Ansprüche zur Verminderung des Haarwuchses.

9. Zusammensetzung gemäß einem der Ansprüche 1-7 zur Verwendung bei der Vorbeugung und/oder Behandlung von Hirsutismus, wobei die Verwendung die topische Anwendung der Zusammenfassung umfasst.

10. Pharmazeutische oder kosmetische Zusammensetzung oder medizinische Vorrichtung, umfassend die Zusammensetzung gemäß einem der Ansprüche 1-7.

## Revendications

1. Composition comprenant au moins un excipient et/ou additif technologique pharmaceutiquement acceptable et/ou de qualité cosmétique et/ou de qualité alimentaire et un mélange qui comprend ou, en variante, consiste en, au moins les composants suivants :
(a) au moins une isoflavone ;
(b) au moins un stérol ; et
(c) de l'apigénine.

2. Composition selon la revendication 1, dans laquelle l'au moins une isoflavone (a) est extraite d'au moins une plante appartenant à la famille Fabaceae, de préférence appartenant à l'un des genres *Glycine, Phaseolus, Medicago, Vigna, Pueraria* et/ou *Trifolium,* mieux encore appartenant à l'espèce *Glycine max* ou *Glycine soja.*

3. Composition selon la revendication 1 ou 2, dans laquelle l'isoflavone (a) est la génistéine, la daidzéine, la glycitéine et leurs combinaisons, et/ou le stérol (b) est le β-sitostérol, le cycloarténol et/ou le stigmastérol et leurs combinaisons.

4. Composition selon la revendication 1, 2 ou 3, dans laquelle l'apigénine (c) est extraite d'au moins une plante des genres *Matricaria, Chamaemelum, Apium, Petroselinum, Camellia* et/ou *Citrus.*

5. Composition selon l'une des revendications précédentes, dans laquelle (a) est présente en une quantité de 0,05 % à 10 % et/ou (b) est présent en une quantité de 0,1 à 10 % et/ou (c) est présente en une quantité de 0,1 à 10 %, dans laquelle toutes les quantités se réfèrent au poids du composant par rapport au poids total de la composition.

6. Composition selon l'une des revendications précédentes, sous la forme d'une crème, d'une pommade, d'un gel, d'une solution, d'une suspension, d'une émulsion, d'une poudre, d'un spray, d'une gaze, d'un timbre transdermique ou d'autres formes de délivrance convenant pour un usage topique.

7. Composition selon les revendications 1 à 6, comprenant les composants suivants :
a)
| *Principe actif* | *Concentration en poids*/*poids total* |
|---|---|
| Stérol ou mélange de stérols | 2 à 3 % |
| Apigénine | 1,5 à 2,5 % |
| Isoflavones | 0,25 à 0,75 % |
ou, en variante,
b)
| *Principe actif* | *Dose quotidienne* |
|---|---|
| Stérol ou mélange de stérols | 2 à 3 % (de préférence 2,5 %) |
| Apigénine | 1 à 2 % (de préférence 1,5 %) |
| Isoflavones | 0,25 à 0,75 % (de préférence 0,5 %) |
ou, en variante,
c)
| *Principe actif* | *Dose quotidienne* |
|---|---|
| Stérol ou mélange de stérols | 2 à 3 % (de préférence 2,5 %) |
| Apigénine | 0,1 à 1 % (de préférence 0,5 à 0,8 %) |
| Isoflavones | 0,25 à 0,75 % (de préférence 0,5 à 0,6 %) |

8. Utilisation non thérapeutique de la composition de l'une quelconque des revendications précédentes pour réduire la pousse des poils.

9. Composition selon l'une quelconque des revendications 1 à 7, pour une utilisation dans la prévention et/ou le traitement de l'hirsutisme, dans laquelle ladite utilisation comprend l'application topique de ladite composition.

10. Composition pharmaceutique ou cosmétique ou dispositif médical comprenant la composition de l'une des revendications 1 à 7.
